# EUROPEAN PATENT APPLICATION

(11) **EP 0 558 316 A1**
(43) Date of publication of application: **01.09.1993**
(21) Application number: 93301406.0
(22) Date of filing: 25.02.1993
(51) Int. Cl.: H01F 38/14, H04B 5/00, A61B 17/36, H02J 17/00

(54) **An inductive loop power transmission system**

(30) Priority: 27.02.1992 GB 9204200
(71) Applicant: G2 DESIGN LIMITED, Cardiff, CF3 OLT, Wales (GB)
(72) Inventor: Goble, Nigel Mark, Castleton, Nr. Cardiff (GB); Goble, Colin Charles Owen, Canton, Cardiff, CF5 4HJ (GB)
(74) Representative: Blatchford, William Michael

(57) **Abstract**

An inductive loop power transmission system intended primarily for wireless electrosurgical use has a transmitter including a radio frequency power oscillator with a resonant output circuit coupled to a loop antenna, and a receiver with a receiving antenna (L1a) and a power output (L1b) for an electrosurgical appliance. In the receiver, a signalling circuit (Cp, D1, D2, Ct, R3, M1) produces a pulse signal which is representative of the power received by the receiver insofar as the repetition rate of the pulses is generally proportional to the average power level. This pulse signal is used periodically and momentarily to detune a resonant antenna circuit (L1a, Cr, Cd) of the receiver, causing corresponding alterations in the loading of a resonant antenna of the transmitter. These alterations in transmitted power are detected in the transmitter and used to vary the transmitter output power so as to maintain the receiver power level at the receiver substantially constant.

## Description

This invention relates to an inductive loop power transmission system which has particular application in the field of cordless devices for use in an operating theatre.

It is well known that electrical power can be supplied to a portable device by coupling an alternating current power source to a transmitter coil which induces an alternating current in a receiver coil in the portable device. This inductive loop technique is generally used at radio frequencies, but often is precluded by the need to limit radio frequency (RF) emissions to avoid interference to other RF services.

In the field of surgical operations, a complicated invasive operation may require the use of a considerable number of electrically powered surgical tools and other devices. Since a sterile operating field must be maintained within an operating theatre, these tools and devices together with their cables to the main supply must be capable of being sterilised. Often, the equipment requires control units to be positioned outside the sterile field and to be operated manually by an independent operator acting on instructions from the surgical team, since mains operated control units cannot be sterilised to a sufficient degree to allow operation by users who are also in contact with the patient. The margin for the sterile field is drawn at the cable to operating table connection. All such connecting leads from the operating table have to be autoclaved by steam autoclaves which operate at temperatures from 115°C to 134°C for cycle durations of 30 minutes and 3.5 minutes respectively. Repeated sterilisation accelerates the ageing effects on the connectors and leads. Consequently much of operating theatre equipment failure is due to failure of these components.

RF emission standards are relaxed for operating theatres, particularly since electrosurgery equipment, by its very nature, results in high levels of RF emission. An inductive loop power source may, therefore, be used at significant power levels.

Such a power source eliminates the requirement for connecting leads yet preserves the integrity of the sterile operating field. However, a difficulty with devices relying on power received from an inductive loop system is that the received power fluctuates widely as a result of varying coupling angles and ranges. This would be a particular disadvantage with surgical tools, since these tend to be used at different angles and moved about to an extent which, for devices requiring significant power, prevents the maintenance of sufficient power levels without using unreasonably powerful transmitting sources.

In order to tackle this problem, the present invention, according to a first aspect thereof, provides an inductive loop power transmission system having a transmitter and a receiver, wherein the receiver includes signalling means responsive to the level of received power for providing a feedback signal, and wherein the transmitter has a detector for detecting the feedback signal and a power control circuit responsive to the detected signal to adjust the output power of the transmitter thereby to compensate for changes in the efficiency of power transmission from the transmitter to the receiver. More particularly, the signalling means are operable periodically and momentarily to alter the power consumption of the receiver from the transmitter according to the received power level, the transmitter detector is operable to monitor the alteration in power delivered to the receiver, and the power control circuit is operable to adjust the output power of the transmitter according to the signalled receiver power consumption as detected by the detector. Signalling may be achieved by means of a circuit in the signalling means for momentarily altering the resonant frequency of a tuned aerial circuit in the receiver so as momentarily to reduce the loading of the transmitter. The received power consumption may be represented by the rate at which these momentary alterations occur, with the transmitter detector providing a detector output signal to the power control circuit which is representative of the rate at which detected alterations in the delivered power occur.

According to another aspect of the invention, a receiver for an inductive loop power transmission system comprises an antenna for receiving a radio frequency power signal transmitted from a transmitter of the system, and signalling means for providing a feedback signal detectable by the transmitter and representative of the level of received radio frequency power. According to yet another aspect of the invention, there is provided a transmitter for an inductive loop power transmission system, in which a feedback signal representative of a received power level is generated by a receiver of the system, comprising a resonant radio frequency output circuit including a transmission antenna, a detector operable to monitor momentary changes in the radio frequency power output of the output circuit to produce a detected feedback signal, and a power control circuit responsive to the detected feedback signal to adjust the output power of the output circuit.

The preferred transmitter and the receiver both employ tuned circuits for achieving good magnetic field coupling efficiency. When the receiver is loaded due, for example, to operation of its associated surgical tool, a reduction in the transmitter antenna loop impedance occurs. Similarly if the tuning of the receiver aerial circuit is altered so that it is out of tune with respect to the transmitter, then there is an increase in the transmitter loop impedance. Power feedback control is then possible by providing the above-mentioned repetitive detune within the receiver, the repetition rate being proportional to received power. This may be achieved by altering an inductance or capacitance of the tuned receiver aerial circuit.

The detune repetition rate may be determined by a pulse generator in the form of a charge pump, timing capacitor and threshold detector, one of the tuning components of the tuned aerial circuit being altered on each discharge of the pumped timing capacitor. If the transmitter circuitry reacts by altering the transmitted power level to maintain the received power level constant, linearity of detune repetition rate as a function of received power is unimportant, as the rate of impedance alterations at the transmission loop will always be controlled about a threshold value.

The circuitry required within the receiver may be extremely simple, and may be fabricated to be compatible with standard sterilisation techniques by, for instance, extensive use of ceramic components.

Optimum transmission frequencies are coincidentally those of radio frequency (RF) electrosurgery equipment, which means that the received power can be used directly for application to patient tissue e.g. for RF electrosurgical cutting or coagulation. Other uses of the invention include powering intra-cavitary lighting or piezo motors to provide mechanical assistance, which may be of particular use in minimally invasive surgical procedures. The ability of a power transmission system in accordance with the invention to maintain constant received power allows such devices to be satisfactorily powered largely irrespective of movement within a given range, particularly movements produced by respiration or insufflation of the abdominal cavity with carbon dioxide.

An advantageous feature of the preferred embodiment of the invention is the ability to select receiver power level settings without impairing the ability to sterilise the receiver. This is achieved by arranging for an inductive or capacitive coupling component in the received power level sensing circuitry, the component including a movable element which is outside the main body of the receiver so that the value of the inductance or capacitance can be altered without the need for a mechanically movable connection extending between the outside and the interior of the receiver body.

The invention will now be described by way of example with reference to the drawings in which:-
Figure 1 is a circuit diagram of a receiver for use in an inductive loop power transmission system; and
Figure 2 is a block diagram of a suitable transmitter.

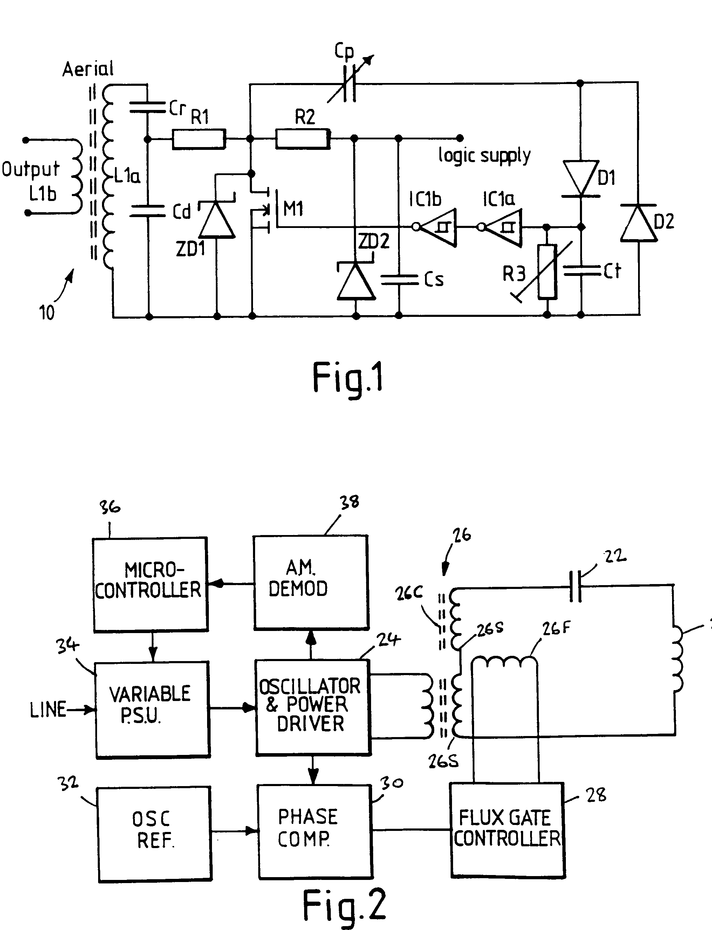

Referring to Figure 1, a receiver for receiving power via an inductive loop link includes a ferrite-cored aerial having a power winding L1b and a tuning winding L1a, the tuning winding L1a forming part of a parallel resonant circuit with the serial combination of two tuning capacitors Cr and Cd. In this embodiment, the aerial has a single pair of windings mounted on a single core so that the aerial has a single axis. However, greater coupling angle immunity can be achieved by employing an aerial with three cores all arranged perpendicularly to each other on x, y, and z axes with each of the windings L1b and L1a shown in Figure 1 split into three portions, one portion of each winding being on a respective one of the three cores.

An alternating current at the transmission frequency developed in winding L1b may be used directly for application to patient tissue in, for example, RF electrosurgery, or it may be rectified to provide a DC supply for other uses.

Coupled across one of the tuning capacitors Cd is a MOSFET M1 in series with a current limiting resistor R1. A protection zener diode ZD1 is connected between the drain and source terminals of the MOSFET M1.

MOSFET M1 is normally switched off. Its gate connection is fed from a pair of Schmitt trigger inverters IC1a and IC1b which act as a threshold detector for a charge pump timing circuit powered via current limiting resistor R1. A variable coupling capacitor Cp is used to charge pump a timing capacitor Ct via diodes D1 and D2 in a conventional manner. Resistor R3 coupled across timing capacitor Ct is a variable factory-set bleed resistance. The power supply for the Schmitt trigger circuits is also obtained via resistor R1 as a DC voltage which is shunt-regulated and smoothed by the combination of resistance R2, zener diode ZD2, and smoothing capacitor Cs.

The resonant components of the aerial circuit are winding L1a, and capacitors Cr and Cd. MOSFET M1 is normally switched off. In resonance, the voltage available across these components typically reaches 1kV rms at maximum power. At a minimum power setting, this voltage level may fall to 200V, corresponding to a minimum to maximum power level ratio of 1 to 25.

The charge pump circuitry together with the MOSFET M1 used as a switching device constitute a signalling circuit for repeatedly and momentarily detuning the aerial circuit in response to the level of received power. Since the operating Q's of the receiver aerial circuit and the transmitting loop of the transmitter (to be described below) have to be maintained well above 50 for efficient power coupling, the amount of detuning required in the receiver to produce a significant change in received power is small. A 1% shift in the resonant frequency of the aerial circuit typically results in a 3dB power dip. Detuning is brought about by effectively shorting out capacitor Cd using MOSFET M1. To keep the maximum voltage drop appearing across MOSFET M1 to reasonable levels, capacitor Cd may be made much larger than capacitor Cr. For a 3dB power dip, the ratio of Cd to Cr may be 50:1. This means that at full power, the rms voltage across MOSFET M1 would be limited to 20V.

The parasitic MOSFET junction diode and the protection zener diode ZD1 act to shunt-rectify the voltage across the capacitor Cd, to produce an average DC voltage at the drain of MOSFET M1 in the region of 4 to 20V. This voltage, available at the drain node, is used to power the Schmitt trigger logic gates IC1a and IC1b via regulating components R2, ZD2, and Cs. Also connected at this drain node is the capacitor Cp which is variable to provide power control. Capacitor Cp is used to charge pump the timing capacitor Ct via diodes D1 and D2. Since bleed resistor R3 is presettable, the charge rate may be factory set prior to potting of the receiver. In operation of the charge pump circuitry, when the voltage on the timing capacitor Ct reaches the upper threshold of the Schmitt trigger gate IC1a, MOSFET M1 is switched on. In the switching transient, capacitor Cd is rapidly discharged of its average DC voltage and the aerial resonant circuit is detuned. When MOSFET M1 is switched on, pumping of the timing capacitor Ct stops and its charge is bled away through resistor R3. Subsequently, the voltage across capacitor Ct reaches the lower threshold of the Schmitt trigger gate IC1a, the MOSFET M1 is switched off, the resonant frequency of the aerial circuit returns to its normal value, and charging of capacitor Ct begins once again.

It will be understood that the rate at which timing capacitor Ct is charged is at least approximately proportional to the level of received power. Consequently, the rate of detuning pulses produced by MOSFET M1 is also proportional to received power.

The relationship between received power and switching rate is adjustable by adjusting capacitor Cp. The two plates of the capacitor Cp are formed as a series of regularly spaced apart pairs of adjacent plates arranged on either side of a groove in the receiver casing so that the plates lie in two parallel planes and are completely isolated from the exterior of the receiver by the solid potting of the whole of the receiver circuitry. The capacitance value of capacitor Cp is then adjusted by means of a potted metallic slider containing a series of small metal plates arranged along a line in a common plane between the above-mentioned two planes and spaced apart at regular intervals at the same pitch as the pitch of the series of pair of plates just mentioned, so that each small metal plate moves between the plates of a respective one of the pairs as the slider moves longitudinally in the groove, i.e. in a direction parallel to the common plane. Movement of the slider alters the air space of each capacitor plate pair. The slider is completely removable for cleaning and sterilisation purposes.

Due to transmitter control lag, when the receiver is taken off load (i.e. the output winding L1b is no longer loaded), the resonant voltage across capacitor Cd rises rapidly. This unwanted energy is dissipated by the protection zener diode ZD1 for the duration of the control delay.

Referring now to Figure 2, which shows the transmitter in simplified form, a transmitter loop antenna 20 is coupled via a coupling capacitor 22 to the output of a fixed frequency power oscillator comprising an oscillator and power driver stage 24 and an output transformer 26.

The transmitter antenna loop is flexible to allow it to be placed around the operating site. Although the inductance of the transformer secondary windings 26S is maintained comparatively high with respect to that of the antenna loop 20 to minimise the effect of loop deformation on resonant frequency, compensation for changes in antenna inductance can be performed by means of a flux gate winding 26F forming part of the transformer 26. The secondary winding 27S is split into two parts which are loosely coupled via a ferrite core 26C. The flux gate winding 26F oriented perpendicularly with respect to the core 26C is connected to a flux gate controller 28 for generating a direct current in the winding 26F which shifts the operating point of he transformer on the B/H (magnetic flux/magnetic field intensity) curve of the ferrite material of the core 26C. Since the B/H curve is non-linear the effective permeability of the ferrite changes according to the magnitude of the flux gate current in winding 26F. Consequently this DC bias may be used to control the mutual coupling between the two sections of the secondary winding 26S. The whole oscillator and power driver is driven as a self-tuned oscillator and therefore assumes a frequency which corresponds to the resonant frequency of the parallel resonant circuit formed by the secondary winding 26S, the transmitting loop antenna 20 and the coupling capacitor 22. The frequency is compared in a phase comparator 30 with a reference frequency generated by a crystal controlled reference oscillator 32 in order to adjust the flux gate DC bias, and hence to maintain the frequency constant at the resonant frequency of the receiver tuned antenna circuit. An alternative technique makes use of a variable capacitor (e.g. a motor driven air-spaced capacitor) in the resonant circuit.

The oscillator and power driver stage 24 is powered from a variable output switched mode power supply unit 34 which is, in turn, controlled by a microprocessor controller 36 responsive to an output signal from an AM (amplitude modulation) demodulator circuit 38.

The demodulator 38 is operable to monitor the current drain of the power oscillator which acts as a feedback signal containing pulses produced by the periodic detuning of the receiver aerial circuit as described above. The pulsed current drain signal is demodulated by the detector, and the pulse repetition rate is monitored by the microcontroller 36. By altering the power output of the power oscillator using the variable power supply unit 34, the demodulated pulse repetition rate can be maintained constant (due to the received power level in the receiver also being maintained constant) thereby compensating for changes in the coupling efficiency between the transmitter loop and the receiver aerial circuit. As an alternative the output voltage of the oscillator can be monitored as a measure of output power.

When the transmitter is activated, the microprocessor controller 36 is programmed to decrease the power in the antenna loop 20 to a standby level when the receiver is taken out of range of the loop 20. Control of full power is then re-established when the receiver is brought back into range (the standby power level being sufficient to cause the receiver, when in its unloaded condition, to produce detuning pulses). The microcontroller 36 also monitors safety features such as quiescent power drain and operating frequency to ensure continuity of loop insulation and to prevent misuse. The transmitter may provide visual indications of coupling efficiency to aid the operator.

## Claims

1. An inductive loop power transmission system having a transmitter and a receiver, characterised in that the receiver includes signalling means responsive to the level of received power for providing a feedback signal, and in that the transmitter has a detector (38) for detecting the feedback signal and a power control circuit (34, 36) responsive to the detected signal to adjust the output power of the transmitter thereby to compensate for changes in the efficiency of power transmission from the transmitter to the receiver.

2. A system according to claim 1, characterised in that the receiver signalling means are operable periodically and momentarily to alter the power consumption of the receiver from the transmitter according to the received power level, the transmitter detector (38) is operable to monitor the alteration in power delivered to the receiver, and the power control circuit (34, 36) is operable to adjust the output power of the transmitter according to the signalled receiver power consumption as detected by the detector (38).

3. A system according to claim 2, characterised in that the receiver signalling means includes a circuit for momentarily altering the resonant frequency of a tuned antenna circuit (L1a, Cr, Cd) of the receiver thereby momentarily to reduce the loading of the transmitter.

4. A system according to claim 3, characterised in that the receiver signalling means include a pulse generator operable to convert the received power level to pulses the repetition rate of which varies according to the power level, and in that the transmitter detector (38) is arranged to produce a detector output signal for feeding to the power control circuit (34, 36) which is representative of the pulse repetition rate.

5. A system according to claim 4, characterised in that the pulse generator comprises a charge pump (Cp, D1, D2), a timing capacitor (Ct), and a threshold detector (IC1a).

6. A system according to claim 4 or claim 5, characterised in that the charge pump includes a variable capacitor (Cp).

7. A system according to claim 6, characterised in that the receiver is potted in an insulative material, and wherein the variable capacitor (Cp) comprises capacitor plates adjacent a portion of the exterior surface of the material and a movable element mounted on the exterior surface portion, the movable element being positioned so as to act as an electric field bridge between the capacitor plates.

8. A system according to claim 6, characterised in that the receiver is potted in a receiver casing having a groove, and wherein the variable capacitor (Cp) comprises at least a pair of adjacent capacitor plates arranged on either side of the groove so that the plates are arranged in two parallel planes and are isolated from the exterior of the receiver by the potting of the receiver, the capacitor further comprising a slider movable longitudinally in the groove between the capacitor plates.

9. A system according to any of claims 4 to 8, characterised in that the tuned antenna circuit of the receiver comprises the parallel combination of a coil (L1a) and at least two capacitors (Cr, Cd) connected in series, and wherein the signalling means includes a switching device (M1) coupled across one of the capacitors (Cd) and actuable in response to pulses from the pulse generator to alter the effective capacitance connected in parallel with the coil (L1a).

10. A system according to any of claims 2 to 9, characterised in that the transmitter comprises a power oscillator (24) having an output transformer (26) with a primary winding and a secondary winding (26S), a loop antenna (20) coupled to the secondary winding (26S), and a capacitor (22) for resonating the secondary winding (26S) and the loop antenna (20) at a predetermined transmission frequency, and in that the detector (38) is arranged to monitor the output voltage of the oscillator (24) as a measure of the instantaneous output power.

11. A system according to any of claims 2 to 9, characterised in that the transmitter comprises a power oscillator (24) having an output transformer (26) with a primary winding and a secondary winding (26S), a loop antenna (20) coupled to the secondary winding (26S), and a capacitor (22) for resonating the secondary winding (26S) and the loop antenna (20) at a predetermined transmission frequency, and in that the detector (38) is arranged to monitor the current drain of the oscillator (24) as a measure of the instantaneous output power.

12. A system according to claim 11, characterised in that the transmitter includes a power supply unit (34) from which the oscillator (24) is powered and a microprocessor controller (36) responsive to an output signal from the detector (38) and operable to alter the output of the power supply unit (34) thereby to adjust the oscillator power output and to maintain the average power consumption of the receiver substantially constant.

13. A receiver for an inductive loop power transmission system comprising an antenna for receiving a radio frequency power signal transmitted from a transmitter of the system, and signalling means for providing a feedback signal detectable by the transmitter and representative of the level of received radio frequency power.

14. A receiver according to claim 13, characterised in that the signalling means are operable periodically and momentarily to alter the instantaneous power consumption of the receiver according to the received power level.

15. A receiver according to claim 14, characterised in that the antenna is part of a tuned antenna circuit (L1a, Cr, Cd) and the signalling means includes a circuit for momentarily altering the resonant frequency of the tuned circuit.

16. A receiver according to claim 15, characterised in that the signalling means include a pulse generator operable to convert the received power level to pulses having a repetition rate which varies according to the power level.

17. A receiver according to claim 16, characterised in that the pulse generator comprises a charge pump (Cp, D1, D2), a timing capacitor (Ct), and a threshold detector (IC1a).

18. A receiver according to claim 16 or claim 17, characterised in that the charge pump includes a variable capacitor (Cp).

19. A receiver according to claim 16, characterised in that the circuit for momentarily altering the resonant frequency of the tuned circuit (L1a, Cr, Cd) includes switching means (M1, ZD1) responsive to the pulse generator and connected to switch in and switch out one of a plurality of capacitors (Cd) forming part of the tuned circuit.

20. A receiver according to claim 19, characterised in that the capacitors (Cr, Cd) are connected in series and the switching means (M1, ZD1) are coupled across the said one capacitor (Cd), and in that the switching means (M1, ZD1) are conductive in one direction during their off state thereby to provide a DC voltage across the said one capacitor (Cd) for powering the signalling means.

21. A transmitter for an inductive loop power transmission system in which a feedback signal representative of a received power level is generated by a receiver of the system, characterised by a resonant radio frequency output circuit (24, 26, 20, 22) including a transmission antenna (20), a detector (38) operable to monitor momentary changes in the radio frequency power output of the output circuit to produce a detected feedback signal, and a power control circuit (34, 36) responsive to the detected feedback signal to adjust the output power of the output circuit.

22. A transmitter according to claim 21, characterised in that the output circuit (24, 26, 20, 22) forms part of a power oscillator and comprises a transformer (26) with a primary winding and a secondary winding (26S), a loop antenna (20) coupled to the secondary winding (26S), and a capacitor (22) for resonating the secondary winding (26S) and the loop antenna (20) at a predetermined transmission frequency, and in that the detector (38) is arranged to monitor the current drain of the oscillator as a measure of instantaneous output power.

23. A transmitter according to claim 22, characterised by a power supply unit (34) from which the oscillator draws its current, and a microprocessor controller (36) responsive to an output signal from the detector (38) and operable to alter the output of the power supply unit (34) thereby to adjust the oscillator power output.

24. An electrosurgical instrument including an inductive loop power transmission system according to any of claims 1 to 12.

25. An electrosurgical instrument including a power receiver unit according to any of claims 13 to 18, the unit being a potted sterilisable assembly.
